(19) Europäisches Patentamt European Patent Office Office européen des brevets

(11) **EP 4 527 421 A1**

(12) **EUROPEAN PATENT APPLICATION**

(43) Date of publication:
**26.03.2025 Bulletin 2025/13**

(21) Application number: **24461623.1**

(22) Date of filing: **25.09.2024**

(51) International Patent Classification (IPC):
***A61L 27/44*** *(2006.01)*        ***A61L 27/52*** *(2006.01)*
***A61L 27/54*** *(2006.01)*

(52) Cooperative Patent Classification (CPC):
(C-Sets available)
**A61L 27/446; A61L 27/52; A61L 27/54;**
A61L 2300/252; A61L 2300/43; A61L 2430/02
(Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **25.09.2023 PL 44621723**

(71) Applicant: **UNIWERSYTET JAGIELLONSKI
31-007 Kraków (PL)**

(72) Inventors:
• **Lewandowska-Lancucka, Joanna
32-010 Sadów (PL)**
• **Krajcer, Aleksandra
31-035 Kraków (PL)**
• **Hinz, Alicja
31-201 Kraków (PL)**
• **Bzowska, Monika
30-348 Kraków (PL)**

(74) Representative: **Witek, Rafal
WTS Patent Attorneys
Witek, Sniezko & Partners
Ul. Rudolfa Weigla 12
53-114 Wroclaw (PL)**

(54) **COMPOSITION CONTAINING A BIOACTIVE HYDROGEL AND A METHOD OF OBTAINING THEREOF**

(57)    The disclosed invention relates to a bioactive hybrid system based on a collagen/chitosan/modified hyaluronic acid hydrogel (ColChHAmod) with insulin (INS)-functionalized silica particles attached to the matrix and a method for preparation thereof. The resulting material is suitable for use in regenerative medicine, particularly for the restoration of dental bone defects.

EP 4 527 421 A1

(52) Cooperative Patent Classification (CPC): (Cont.)

C-Sets
**A61L 27/446, C08L 5/08;**
**A61L 27/446, C08L 89/06**

**Description**

The presented invention relates to a bioactive hybrid system based on a collagen/chitosan/modified hyaluronic acid (ColChHAmod) hydrogel with insulin (INS)-functionalized silica particles attached to the matrix, and a method for preparation thereof. The resulting material is suitable for use in regenerative medicine, particularly for the restoration of dental bone defects.

Scaffolds for cells used in regenerative medicine should be bioactive and have the ability to interact with surrounding tissues. For example, immobilization of drugs, growth factors (i.e. TGF-$\beta$, FGFs, PDGFs, IGF I-II, bone morphogenetic protein (BMP-2)) is used to give the scaffold osteoinductive properties. BMP-2 protein, despite its good therapeutic effects, has many side effects, such as short half-life or rapid enzymatic degradation and inactivation under physiological conditions. One of its substitutes is insulin (INS) and insulin-like growth factors (IGF-1), which promote osteogenesis and angiogenesis and have the ability to promote vascularization and provide nutrients for bone formation.

Patent document EP2396046 describes synthetic monetite matrices that are improved by the incorporation of bioactive calcium and silicon compounds or other pharmacological substances (e.g. insulin-like growth factor, antibiotics, antiinflammatory and anticancer agents, bisphosphonates), affecting the degradation time of the obtained material, promote bone regeneration and improve their osteoinductivity, osteoconductivity and biomechanical properties.

Patent document US20220111119 describes a therapeutic hydrogel with hyaluronic acid for regeneration, reconstruction of tissues whose structure has been disrupted. A topical injection of sterile therapeutic biomimetic hyaluronic acid with a tissue reconstructing agent (THC and/or any other reduced metabolite of THC, namely di-, hexa- or octahydrocurcuminoids) and a tissue regenerating agent (e.g. bone morphogenetic protein, insulin-like growth factor or other cellular factors such as stem cells, fibroblasts) is proposed.

Patent document EP2956185 describes a polymeric system for releasing an active substance (e.g. insulin). The solution relates to an injectable hydrogel containing a polymeric system as a carrier for the delivery of a biologically active substance or drug. A first polymeric phase containing the active substance is dispersed within a second polymeric phase containing a cross-linked polymer-phenol conjugate.

The invention disclosed in EP2529764 relates to a biodegradable composite material, in particular a bone-forming material, with improved properties. The system includes an inorganic component (e.g. bioceramics, in particular calcium phosphate ceramics, bio-glasses containing $SiO_2$, $Na_2O$, $K_2O$, CaO and/or $P_2O_5$ glass ceramics or mixtures thereof) and an organic component (i.e. polyesters, lactic acid or glycolic acid polymers, gelatin, collagen, glycosaminoglycan, hyaluronate, sodium hyaluronate, calcium hyaluronate, methyl cellulose, ethyl cellulose, propyl cellulose, hydroxypropyl cellulose, hydroxyethyl cellulose, starch, dextran, hydroxyethyl starch, alginate, polyethylene glycol, albumin, chitosan, polypeptides, proteins and antibiotics). The general steps for obtaining the known material are adding at least one organic component to a solution containing water to form a suspension; adding inorganic components and possibly a crosslinking agent to the suspension and mixing the components to form a solution. The final step is drying the mixture by freeze-drying to form the composite material.

The inventors' earlier publication "Bioactive injectable composites based on insulin-functionalized silica particles reinforced polymeric hydrogels for potential applications in bone tissue engineering". Journal of Materials Science & Technology 105 (2022) describes a method to immobilize insulin to amine group-functionalized silica particles (two variants of insulin attachment by electrostatic interaction or covalent bonding). The preparation of a hybrid material in injectable form (collagen/chitosan/modified hyaluronic acid with insulin-functionalized silica particles attached to the matrix) and genipin crosslinking are also presented.

Osteoporosis and other civilization diseases (e.g. caries, cancer) contribute to many dental conditions, including jawbone defects following tooth extraction, post-accident defects or resections in the case of neoplasms. Reconstructive materials currently used to restore bone defects are either own (autogenous) bone or bone substitute grafts (e.g. ground bone with hydroxyapatite).

Consequently, new bone substitutes or multifunctional biomaterials with specific properties are being sought. Currently, the most commonly used reconstructive material for the reconstruction of bone defects in dentistry is own (autogenous) bone or bone substitute grafts (e.g. ground bone with hydroxyapatite). Unfortunately, the material taken from the patient is often insufficient for reconstruction and constitutes an extra burden on the body. Therefore, new bone substitutes, multifunctional biomaterials with specific properties (biocompatible, resorbable, bioactive) are being sought for prosthetics and implants in regenerative dentistry.

In addition to solving the above problems, the specific aim of the invention is to provide a biomaterial with reduced swelling. Particularly in the case of materials intended for use in regenerative medicine, especially for the restoration of dental bone defects, this feature is of great practical importance, and the excessive swelling of known materials limits their actual use in dentistry.

Unexpectedly, the aim stated above has been achieved in the present invention.

The subject of the invention is the composition and the method for preparation thereof defined in the appended claims.

The materials developed according to the invention can be used especially for the reconstruction of small-sized bone defects, including jawbone defects following tooth extractions, post-accident defects and resections in the case of neoplasms. The material may also offer a promising, less costly alternative/support system for controlled delivery of BMP-2.

Two types of interactions were used to immobilize insulin on the surface of amine-modified silica particles: electrostatic and covalent. In a preferred embodiment, the material obtained according to the invention was prepared in the form of freeze-dried flakes and also in a reference injectable form, the protocol for obtaining which is disclosed in the inventors' publication cited above. In-depth physicochemical characterization and a panel of *in vitro* biological assays using stem cells were carried out. Especially in their freeze-dried form, the materials of the invention show application potential as dental materials. Unexpectedly, they are characterized by a significant reduction in the swelling ratio (almost eight times as compared to the injectable form known from the state of the art). Thus, the obtained lyophilizate, once positioned in the space of the treated defect, could effectively fill it by creating a scaffold for cell attachment while minimizing the expansion of the material due to swelling. In addition, an experiment conducted under artificial saliva conditions revealed that the freeze-dried systems exhibit stability when in contact with simulated fluid, suggesting that they are fitted for the role of dental materials. Comparing the release profiles for corresponding materials from two different groups, but containing carriers with identically bound insulin (HE vs LE and HC vs LC), a significant delay in substance release was observed for the freeze-dried materials. *In vitro* studies confirmed the absence of hemolytic properties of degradation products formed by the breakdown of hydrogels and lyophilizates. In addition, these products are not toxic to osteoblasts and osteoclasts and stimulate moderately the metabolic activity of hBMMSC (human mesenchymal stem cells) cells. Insulin in the material with a higher concentration of particles (LC 800) had an inhibitory effect on the expression of osteoclast markers. This phenomenon indicates that the therapeutic effect of insulin in the materials may also be related to a decrease in the activity of cells responsible for the resorption process. Analyses with qRT-PCR showed an increase in the hRunx2 transcription factor in hBMMSCs after incubation with insulin-containing test materials. The hRunx2 transcription factor is a component of the signal transduction pathway from insulin, thus this result confirms that insulin immobilized in the materials is active. The properties of the new formulation in the form of lyophilizates, as demonstrated in the course of the studies presented in the application, therefore confirm the unexpected advantages of the developed material as compared with the previously developed injectable form.

For better illustration, figures are attached to this description.

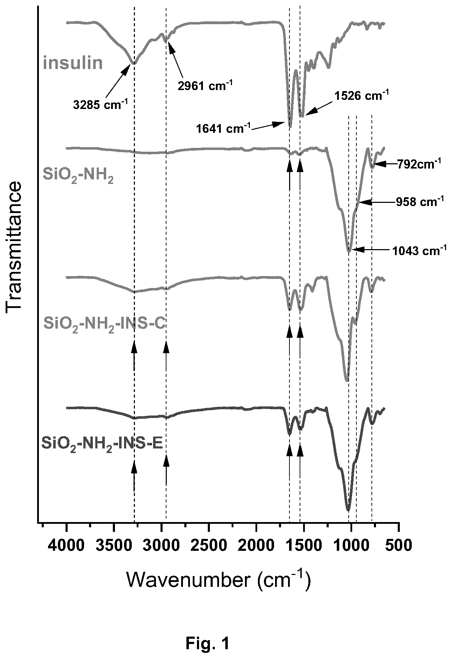

**Fig. 1** shows FTIR spectra for insulin, silica particles containing amine groups ($SiO_2$-$NH_2$) and carriers functionalized with electrostatically ($SiO_2$-$NH_2$-INS-E) or covalently ($SiO_2$-$NH_2$-INS-C) immobilized insulin.

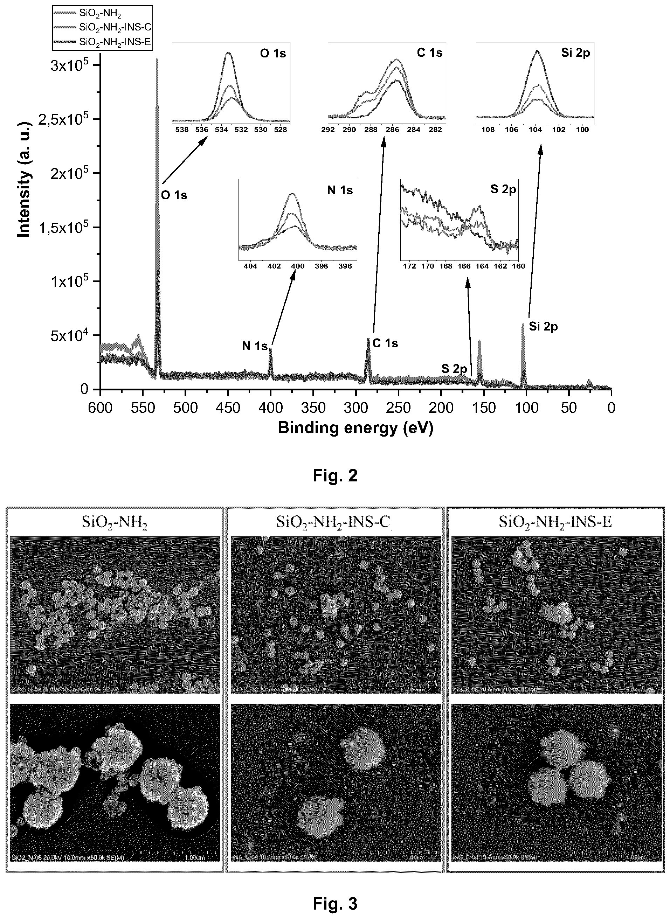

**Fig. 2** shows XPS survey spectra for silica particles and carriers with the protein attached with the two methods, as well as magnifications in the form of high resolution spectra for the elements present in the analyzed samples.

**Fig. 3** shows microphotographs for $SiO_2$-$NH_2$ silica particles and carrier-protein systems in which insulin is bound electrostatically ($SiO_2$-$NH_2$-INS-E) or covalently ($SiO_2$-$NH_2$-INS-C).

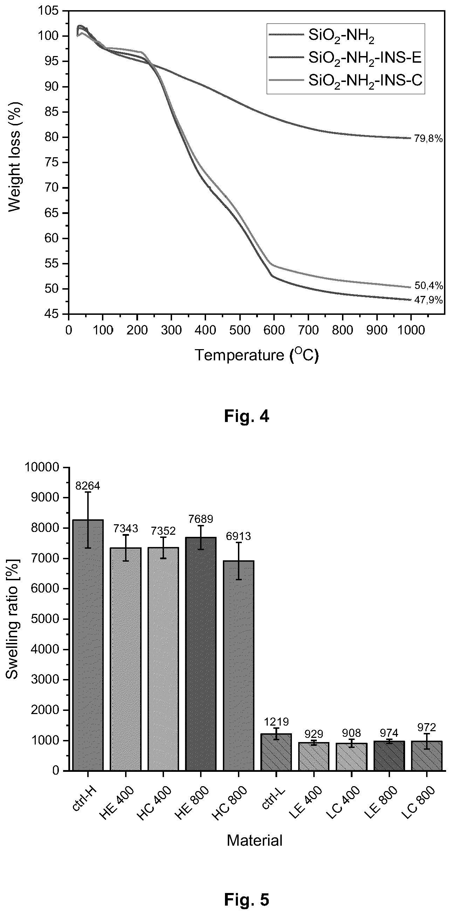

**Fig. 4** shows TG profiles obtained for silica particles ($SiO_2$-$NH_2$) and for silica particles with insulin attached electrostatically ($SiO_2$-$NH_2$-INS-E) or covalently ($SiO_2$-$NH_2$-INS-C).

**Fig. 5** shows swelling ratio for a series of materials from the freeze-dried group (ctrl-L, LE400, LC400. LE800, LC800) and the reference group of injectable hydrogel systems (ctrl-H, HE400, HC400. HE800, HC800).

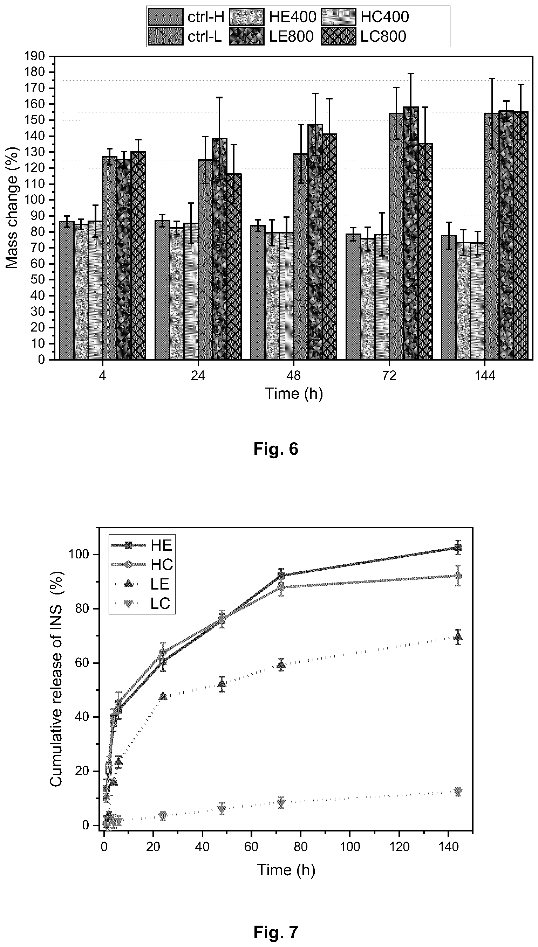

**Fig. 6** shows data on the degradation of the hybrids in the form of freeze-dried flakes and the reference group of injectable hybrid materials conducted in artificial saliva for 144 h.

**Fig. 7** shows the profile of insulin release from the model hybrid systems containing particles with the protein attached electrostatically (HE or LE) or covalently (HC or LC) in the type of hydrogel systems (HE or HC) or lyophilizates (LE or LC).

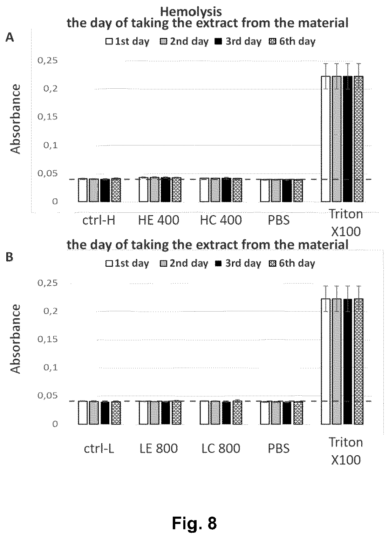

**Fig. 8** shows the effect of degradation products of the tested materials on hemolysis. Human erythrocytes were incubated for 3 hours at 37°C with extracts collected from **A.** hydrogels (ctrl-H, HE 400 and HC 400) or **B.** lyophilizates (ctrl-L, LE 800 and LC 800). PBS was used as a negative control, and Triton X100 was used as a maximum hemolysis control. The level of hemolysis was examined by measuring the absorbance of cyanmethemoglobin obtained in the Drabkin reaction.

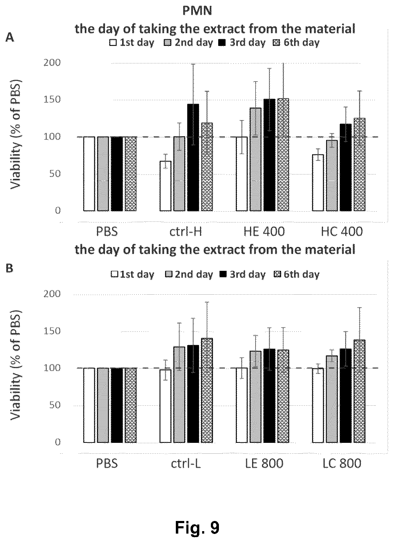

**Fig. 9** shows the evaluation of the effect of the degradation products of the tested materials on human neutrophils (PMNs). PMNs isolated from human peripheral blood were incubated for 24 hours with extracts collected from **A.** hydrogels (ctrl-H, HE 400 and HC 400) or **B.** lyophilizates (ctrl-L, LE 800 and LC 800). PBS buffer was used as a negative control. After the incubation time, ATP levels directly proportional to cell viability were measured in the cells using ATPlite PerkinElmer kit. The result was presented as % of negative control (normal conditions).

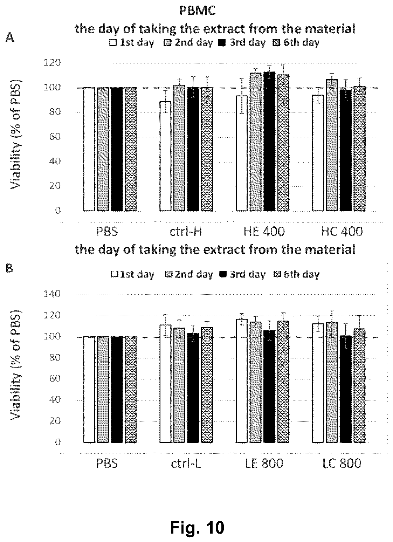

**Fig. 10** shows the evaluation of the effect of degradation products of the tested materials on mononuclear cells isolated from human peripheral blood (PBMC). PBMC cells were incubated for 48 hours with extracts collected from **A.** hydrogels (ctrl-H, HE 400 and HC 400) or **B.** lyophilizates (ctrl-L, LE 800 and LC 800). PBS buffer was used as a negative control. After the incubation time, ATP levels directly proportional to cell viability were measured in the cells using ATPlite PerkinElmer kit. The result was presented as % of negative control (normal conditions).

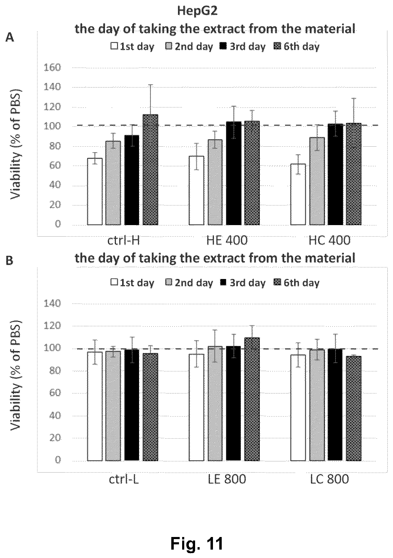

**Fig. 11** shows the evaluation of the effect of the degradation products of the tested materials on a model of human liver cells (HepG2). HepG2 cells were incubated for 48 hours with extracts collected from **A.** hydrogels (ctrl-H, HE 400 and HC 400) or **B.** lyophilizates (ctrl-L, LE 800 and LC 800). PBS buffer was used as a negative control. After the incubation time, the metabolic activity levels directly proportional to cell viability were measured in the cells using MTT assay. The result was presented as % of negative control (normal conditions).

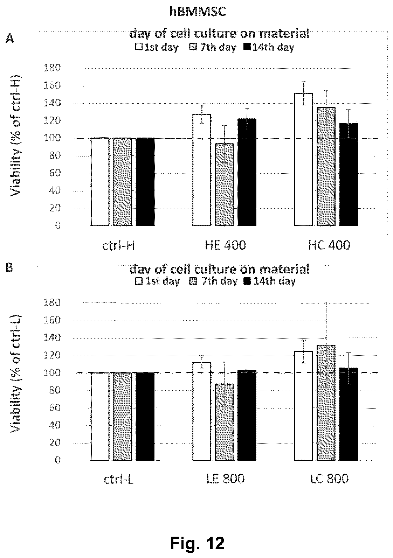

**Fig. 12** shows the evaluation of cytotoxicity of the insulin-containing materials against osteoblast precursors. Human mesenchymal stem cells isolated from bone marrow (hBMMSCs) were incubated for 3, 7 or 14 days with **A.** hydrogels (ctrl-H, HE 400 and HC 400) or **B.** lyophilizates (ctrl-L, LE 800 and LC 800). After the incubation time, metabolic activity levels directly proportional to cell viability were measured in the cells using Invitrogen's alamarBlue assay. The result was presented as % of viability obtained for hydrogel (ctrl-H) or freeze-dried (ctrl-L) control (insulin-free).

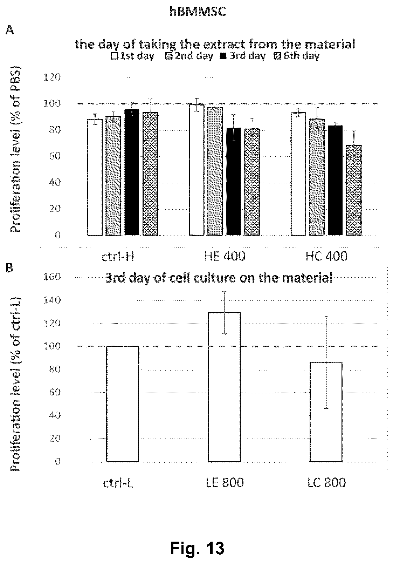

**Fig. 13** shows the evaluation of the effect of the insulin-containing materials on the proliferation of osteoblast precursors. Human mesenchymal stem cells isolated from bone marrow (hBMMSCs) were subjected to **A.** 3-day incubation with extracts harvested from hydrogels (ctrl-H, HE 400 and HC 400) or **B.** 3-day culture in the presence of lyophilizates (ctrl-L, LE 800 and LC 800). After the incubation time, proliferation levels were measured in the cells by DNA staining in the Invitrogen's CyQuant assay. The result was presented as **A.** % of proliferation obtained for the negative control - PBS (normal conditions) or **B.** % of proliferation obtained for the freeze-dried (ctrl-L) control (insulin-free).

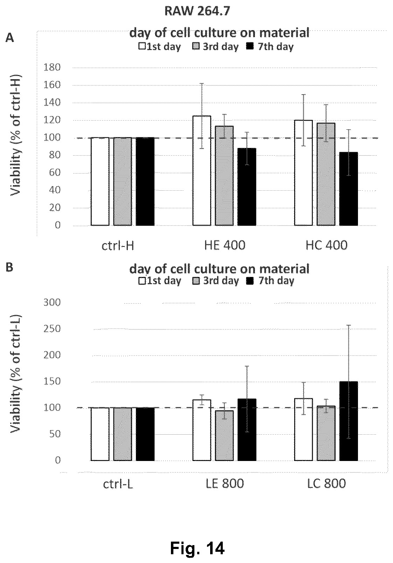

**Fig. 14** shows the evaluation of cytotoxicity of the insulin-containing materials against osteoclast precursors. RAW 264.7 mouse monocyte-macrophage cells were incubated for 1, 3 or 7 days with **A.** hydrogels (ctrl-H, HE 400 and HC 400) or **B.** lyophilizates (ctrl-L, LE 800 and LC 800). After the incubation time, metabolic activity levels directly proportional to cell viability were measured in the cells using Invitrogen's alamarBlue assay. The result was presented as % of viability obtained for hydrogel (ctrl-H) or freeze-dried (ctrl-L) control (insulin-free).

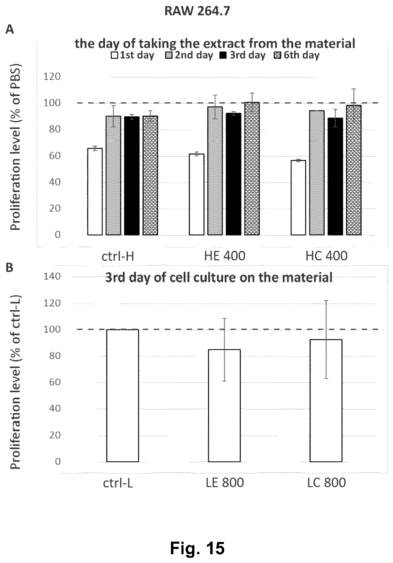

**Fig. 15** shows the evaluation of the effect of the insulin-containing materials on the proliferation of osteoclast precursors. RAW 264.7 mouse monocyte-macrophage cells were subjected to **A.** 3-day incubation with extracts harvested from hydrogels (ctrl-H, HE 400 and HC 400) or **B.** 3-day culture in the presence of lyophilizates (ctrl-L, LE 800 and LC 800). After the incubation time, proliferation levels were measured in the cells by DNA staining in Invitrogen's CyQuant assay. The result was presented as **A.** % of proliferation obtained for the negative control - PBS (normal conditions) or **B.** % of proliferation obtained for the freeze-dried (ctrl-L) control (insulin-free).

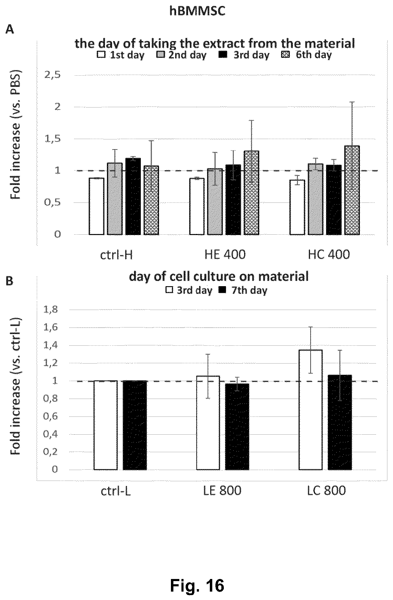

**Fig. 16** shows the evaluation of the effect of the insulin-containing materials on alkaline phosphatase (ALP) activity levels in osteoblast precursors. Human mesenchymal stem cells isolated from bone marrow (hBMMSCs) were subjected to **A.** 7-day incubation with extracts harvested from hydrogels (ctrl-H, HE 400 and HC 400) or **B.** 3 and 7 days culture in the presence of lyophilizates (ctrl-L, LE 800 and LC 800). After the incubation time, the cells were lysed and ALP activity was measured in the presence of p-nitrophenyl phosphate substrate. The absorbance of the resulting enzymatic reaction product was measured at 405 nm. The result was presented as **A.** the multiplicity of the absorbance obtained for the negative control - PBS (normal conditions) or **B.** the multiplicity of the absorbance obtained for the freeze-dried (ctrl-L) control (insulin-free).

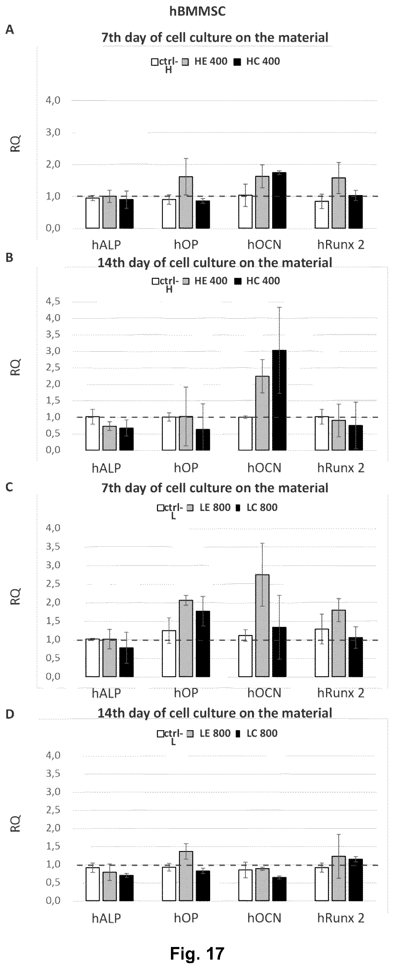

**Fig. 17** shows the evaluation of the effect of the insulin-containing materials on the expression of genes activated during the differentiation of osteoblast precursors. Human mesenchymal stem cells isolated from bone marrow (hBMMSCs) were subjected to **A.** 7-day or **B.** 14-day culture in the presence of hydrogels (ctrl-H, HE 400 and HC 400); **C.** 7-day or **D.** 14-day culture in the presence of lyophilizates (ctrl-L, LE 800 and LC 800). After the incubation time, gene expression analysis was performed in the cells using quantitative polymerase chain reaction (qRT-PCR). Transcript levels were determined for human alkaline phosphatase (hALP), osteopontin (hOP), osteocalcin (hOCN) and transcription factor hRunx2 genes. The RQ parameter expresses the multiplicity of the expression level of a given gene compared to insulin-free ctrl-H or ctrl-L material.

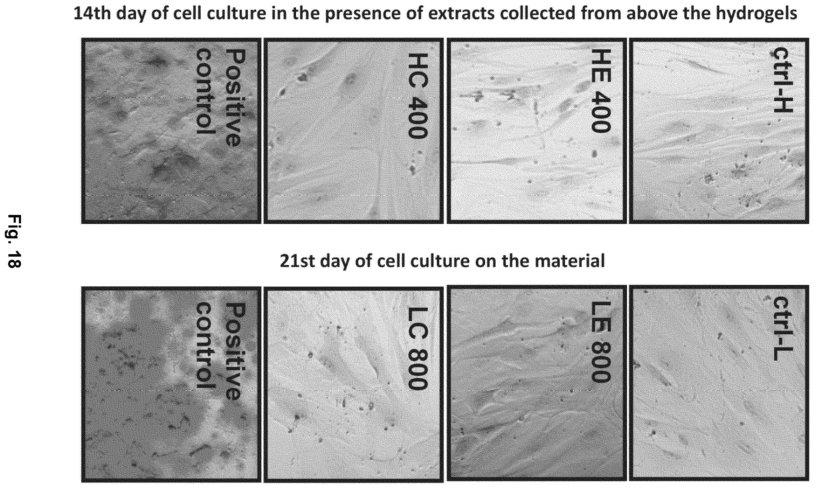

**Fig. 18** shows the evaluation of the effect of the insulin-containing materials on the presence of calcium phosphate crystals produced by osteoblasts. Human mesenchymal stem cells isolated from bone marrow (hBMMSCs) were subjected to 14 days of incubation with extracts harvested from hydrogels (ctrl-H, HE 400 and HC 400) or 21 days of culture in the presence of lyophilizates (ctrl-L, LE 800 and LC 800). After the incubation time, the cells were fixed and stained with alizarin red. The resulting slides were observed under a bright-field microscope. Positive control are conditions under which hBMMSCs differentiate toward osteoblasts. Orange aggregates indicate the presence of calcium phosphate crystals.

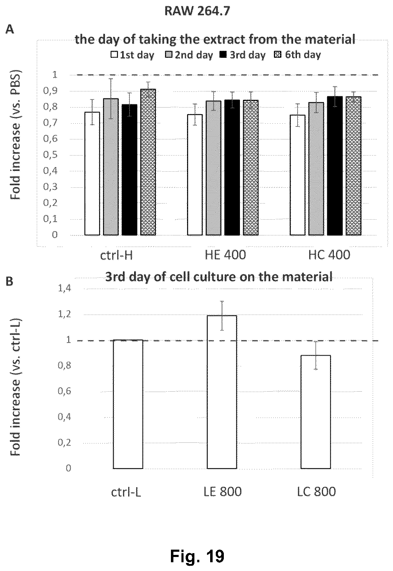

**Fig. 19** shows the evaluation of the effect of the insulin-containing materials on the level of tartrate-resistant acid phosphatase (TRAP) activity in osteoclast precursor cells. RAW 264.7 mouse monocyte-macrophage cells were subjected to **A.** 3-day incubation with extracts harvested from hydrogels (ctrl-H, HE 400 and HC 400) or **B.** 3-day culture in the presence of lyophilizates (ctrl-L, LE 800 and LC 800). After the incubation time, the cells were lysed and TRAP activity was measured in the presence of p-nitrophenyl phosphate substrate. The absorbance of the resulting enzymatic reaction product was measured at 405 nm. The result was presented as **A.** the multiplicity of the absorbance obtained for the negative control - PBS (normal conditions) or **B.** the multiplicity of the absorbance obtained for the freeze-dried (ctrl-L) control (insulin-free).

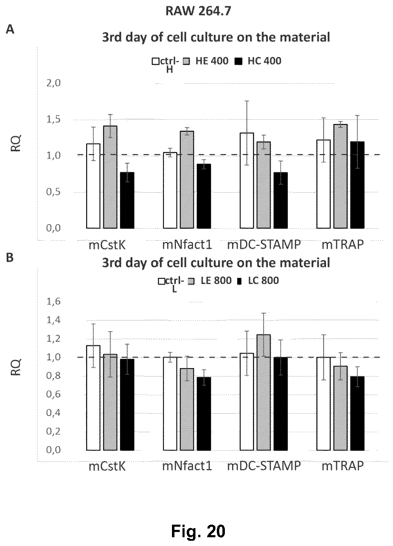

**Fig. 20** shows the evaluation of the effect of the insulin-containing materials on the expression of genes activated during the differentiation of osteoclast precursors. RAW 264.7 mouse monocyte-macrophage cells were subjected to **A.** 3-day culture in the presence of hydrogels (ctrl-H, HE 400 and HC 400) or **B.** 3-day culture in the presence of lyophilizates (ctrl-L, LE 800 and LC 800). After the incubation time, gene expression analysis was performed in the cells using quantitative polymerase chain reaction (qRT-PCR). Transcript levels were determined for mouse cathepsin K (mCtsK), transcription factor mNfatc1, mDC-STAMP fusion protein and tartrate-resistant alkaline phosphatase (mTRAP) genes. The RQ parameter expresses the multiplicity of the expression level of a given gene compared to insulin-free ctrl-H or ctrl-L material.

**Example 1. Preparation of the composition of the invention**

**Synthesis of silica particles functionalized with amine groups**

Silica particles functionalized with amine groups were obtained by a modified Stöber method based on the protocol presented in Lewandowska- Ł ańcucka J *et al.,* International journal of biological macromolecules, 2019, 136, 1196-1208. In brief, 5 mL of deionized water and 5.1 mL of 99% ethanol were mixed and placed on a magnetic stirrer. 1 mL of TEOS (tetraethoxysilane) and 0.1 mL of APTES (aminopropyltriethoxysilane) were dropped into the system. The resulting system was stirred (450 rpm) at room temperature for half an hour. The obtained milky suspension was subjected to several cycles of centrifugation and washing (1 time with ethanol, 3 times with water). After the last centrifugation and removal of the liquid from above the precipitate, the particles were frozen and freeze-dried. A white powder ($SiO_2$-$NH_2$) capable of being suspended in water was obtained. Studies using scanning electron microscopy (SEM) (Fig. 3, $SiO_2$-$NH_2$) indicate that structures were obtained in two size populations - larger, of about 500 nm in diameter, and smaller, measuring about 100 nm.

**Obtaining silica particles with attached insulin $SiO_2$-$NH_2$-INS**

In order to create a system in which amine group-functionalized silica particles would be an INS carrier, two approaches for its attachment were used, which are described in detail in Krajcer A et al., Journal of Materials Science & Technology, 2022, 105, 153-163.

**Electrostatic attachment of insulin, $SiO_2$-$NH_2$-INS-E**

Briefly, the first one involved the use of electrostatic interactions between the particles' amine groups, which were protonated under experimental conditions, and negatively charged insulin fragments.

The previously obtained silica particles (20 mg) were dispersed in 5 mL of 50 mM MES (2-(N-morpholino) ethanesulfonic acid) buffer, pH=5, subjecting the system to pulsed sonication for 40 minutes. The resulting milky suspension was then placed on a magnetic stirrer operating at 250 rpm and 10 mg of insulin dissolved in 2.5 mL of the same MES buffer was added. Another 2.5 mL of MES buffer was introduced into the system, which was used to rinse the vial with the insulin solution in order to thoroughly transfer the protein into the particle suspension. The mixture prepared in this way was left on a magnetic stirrer for 24 hours (300 rpm). After this time, the particles were centrifuged (10 minutes, 9000 rpm) and further centrifuged and washed with distilled water for three times (10 minutes, 9000 rpm). The material ($SiO_2$-$NH_2$-INS-E) was then frozen and freeze-dried. A white powder capable of being suspended in aqueous solutions was obtained.

**Covalent attachment of insulin, $SiO_2$-$NH_2$-INS-C**

The second approach was based on the formation of covalent bonds between the carboxyl groups of the protein and the amine groups of the particles using the initiator chemistry of EDC (1-ethyl-3-(3-dimethylaminopropyl) carbodiimide) and NHS (N-hydroxysuccinimide).

Similar to the approach used above, 20 mg of $SiO_2$-$NH_2$ particles were suspended in 5 mL of 50 mM MES (2-(N-morpholino)ethanesulfonic acid) buffer pH=5 by sonication in pulse mode for 40 minutes. The resulting milky suspension was then placed on a magnetic stirrer, setting the speed to 250 rpm. At this step, the initiators EDC (20 mg) and NHS (12 mg) (EDC/NHS molar ratio was 1:1) and 10 mg of insulin were added sequentially to the system. Each of the three components was introduced as a solution formed by dissolving the respective aliquots (20 mg EDC/12 mg NHS/10 mg INS) in 2 mL of MES buffer for EDC and NHS initiators and 2.5 mL for INS. All solutions were transferred to the particle suspension quantitatively, using additionally 1 mL of MES buffer for each initiator solution added and 2.5 mL for the protein solution. The system was then left on a magnetic stirrer for 24 hours (300 rpm). In the next step, the particles were centrifuged (10 minutes, 9000 rpm) and purified during three cycles of centrifugation-wash with distilled water (10 minutes, 9000rpm). The material in the form of particles after protein immobilization (SiO$_2$-NH$_2$-INS-C) was frozen and freeze-dried. As the final product, a white powder was obtained, which was suspendable in aqueous solutions.

**Preparation of collagen (Coll) solution**

A solution of type I collagen, extracted from the rat tail, in the form of a solution with a concentration in the range of 3.42-4.06 mg/mL in 0.01 N hydrochloric acid (HCl) was purchased from BD Biosciences and was used unchanged by the user.

**Preparation of chitosan (Ch) solution**

A 1% w/w solution of chitosan was obtained by dissolving a weight of chitosan (low molecular weight, Sigma-Aldrich; Mv = 120 kDa, 79% deacetylated), in 1% v/v acetic acid for 24 hours on a magnetic stirrer (600 rpm) at room temperature.

**Preparation of a solution of hyaluronic acid functionalized with amine groups (HAmod)**

The procedure for obtaining lysine-modified hyaluronic acid is presented in publication by Gilarska A et al., (2020), Int. J. Biol. Macromol, 155, 938-950.
MES buffer (50 mM) was prepared by dissolving 0.97 g of 2-(N-morpholino)ethanesulfonic acid (MES) in 100 mL of deionized water and the pH value was set to 4 using 0.1 M NaOH solution. The whole was filtered through a syringe filter. 500 mg of hyaluronic acid (HA) was dissolved in 20 mL of MES buffer (50 mM, pH=4) and then 360 mg of EDC (N-(3-dimethylaminopropyl)-N'-ethylcarbodiimide hydrochloride), 220 mg of NHS (N-hydroxysuccinimide) and 0.73 g of lysine (each reagent was previously dissolved in 5 mL of MES buffer) were added sequentially. The whole mixture was stirred for 24 hours on a magnetic stirrer at room temperature, and then dialyzed for 12 hours into 0.1 M aqueous Na$_2$CO$_3$ solution and successively dialyzed against water for 8 days. The mixture was concentrated on an evaporator and freeze-dried for three days. The degree of lysine substitution of the obtained product (HAmod) was determined by 1H NMR spectroscopy, it was about 25%.

**Preparation of the hydrogel**

By mixing appropriate volumes of biopolymer solutions, i.e. collagen (v=605 $\mu$L, concentration: 3.42-4.06 mg/mL in HCl), chitosan (v=98 $\mu$L, 1% w/w in 1% CH$_3$COOH), functionalised hyaluronic acid (v=147 $\mu$L, 1% w/w in 10x PBS) together with an aqueous dispersion of silica particles with insulin attached in an electrostatic (SiO$_2$-NH$_2$-INS-E; v=100 $\mu$L, concentration=4 mg/mL or 8 mg/mL) or covalent manner (SiO$_2$-NH$_2$-INS-C; v=100 $\mu$L, concentration=4 mg/mL or 8 mg/mL) and genipin solution (v=50 $\mu$L, concentration: 18 mg/mL in 96% EtOH) was obtained as a sol which was then allowed to crosslink for 48 hours at 37°C.
After this time, the resulting hybrid materials were rinsed three times with deionised water and frozen if necessary.

**Preparation of freeze-dried hydrogel**

The obtained hydrogel was subjected to freeze-drying. For this purpose, the rinsed materials were frozen and then placed in the frozen form in a freeze-dryer until the solvents were completely removed under reduced pressure and at a temperature of - 52°C.
The resulting materials are in the form of flakes, the size and thickness of which can be controlled by varying the volume of biopolymer sol poured onto the mould and by selecting the size of the vessel/container in which the sol is placed before the crosslinking process begins.

**Example 2: Characterisation of silica particles with attached insulin SiO$_2$-NH$_2$-INS**

Systems differing in the type of binding of the hormone to the silica particles were obtained - one group of materials contained insulin attached covalently to the carriers (SiO$_2$-NH$_2$-INS-C) and the other via electrostatic interactions (SiO$_2$-NH$_2$-INS-E). The protein content was adjusted by the amount of silica particles carrying biomolecules added to the sol, suspended in deionised water, based on the results of the quantitative Bradford assay. Systems in the form of flakes were created, containing the following concentrations of SiO$_2$-NH$_2$-INS particles: 0.4 mg/mL of sol, for systems with electrostatically attached insulin (LE400) and for covalent immobilisation of the biomolecules (LC400), or 0.8 mg/mL - analogously for particles with two modes of insulin binding, LE800 and LC800. Thus, given the protocols described, the insulin concentrations used were 23 μM (in systems with the lower particle concentrations) and 46 μM (for the systems with double insulin content). As control materials in the form of injectable hydrogels, systems were tested that also contained either 0.4 mg of hormone particles per 1 mL of sol for systems with electrostatically attached protein (HE400) or covalently attached protein (HC400), or the double concentration - 0.8 mg/mL - resulting in the formation of HE800 and HC800 systems. A particle-free matrix (ctrl-H) was used as a reference for materials in the injectable system type, and analogously - lyophilizate without silica carriers (ctrl-L) for the freeze-dried systems. The protocol for the resulting injectable material was presented in our publication (Krajcer A et al., Journal of Materials Science & Technology, 2022, 105, 153-163). The Tango project developed methodologies for obtaining this material in the form of solid freeze-dried flakes. In this protocol, the method for obtaining $SiO_2$-$NH_2$-INS particles is analogous. The difference lies in the preparation of the hybrid material.

The table below **(Table 1)** summarises the names used herein that correspond to the materials obtained.

**Table 1.** Specification of names for the hybrid materials formed

| Hybrid systems with INS attached to $SiO_2$-$NH_2$ particles: | | Control material |
|---|---|---|
| electrostatically | covalently | |
| Hydrogel materials (injectable) | | |
| HE400 | HC400 | ctrl-H |
| HE800 | HC800 | |
| Freeze-dried flakes | | |
| LE400 | LC400 | ctrl-L |
| LE800 | LC800 | |

Hybrid systems based on the biopolymer hydrogel with insulin-functionalized silica particles attached to the matrix (Hyb/$SiO_2$-INS) underwent physicochemical characterisation. Due to literature reports of partial biological inactivation of proteins as a result of their permanent attachment to the carriers, two alternative approaches based on different interactions between silica particles and insulin were tested, including covalent and electrostatic attachment. The efficiency of insulin attachment was verified by FTIR/XPS/TG analyses and also by the Bradford method (UV-Vis spectra measurements). A new formulation of the system was obtained in the form of freeze-dried flakes and also a previously developed injectable formulation as a reference material. Both types of material were subjected to degradation studies under physiological conditions and swelling experiments were performed. Studies of the kinetics of INS release from the developed hybrid materials were also carried out (Bradford spectrophotometric assay).

**1. Characterisation of silica carriers for insulin. Efficiency of protein attachment Fourier Transform Infrared Spectroscopy (FTIR) in the qualitative assessment of insulin attachment**

A qualitative evaluation of the insulin attachment efficiency to silica particles via electrostatic interactions or with the formation of covalent bonds was made by analysing Fourier Transform Infrared Spectroscopy (FTIR) results. **Fig. 1** presents spectra for insulin, silica particles with amine groups ($SiO_2$-$NH_2$) and carriers following electrostatic ($SiO_2$-$NH_2$-INS-E) or covalent ($SiO_2$-$NH_2$-INS-C) attachment.

The significant increase in intensity of the bands at 1641 $cm^{-1}$ and 1526 $cm^{-1}$ compared to the signals on the ($SiO_2$-$NH_2$) spectrum is indicative of the presence of insulin in the carrier-protein systems ($SiO_2$-$NH_2$-INS-E and $SiO_2$-$NH_2$-INS-C), as these oscillations can be attributed to the amide I and II bands, respectively. Furthermore, the weak signals at 2961 $cm^{-1}$ and 3285 $cm^{-1}$, corresponding to the $v$(O-H) stretching vibrations within the carboxyl groups present in the insulin molecules and the signals from the $v$(N-H) vibrations at the numerous amide bonds present in the protein molecules respectively, provide additional confirmation of the successful hormone attachment to the carriers. Further analysis of the spectra also identifies the structure of the silica network, manifested as asymmetric and symmetric stretching vibrations at 1043 $cm^{-1}$ $v_{as}$ ($SiO_2$), 958 $cm^{-1}$ $v_{as}$ (Si-OH) and 792 $cm^{-1}$ $v_s$ ($SiO_2$).

**UV-Vis spectrophotometric quantification using the Bradford method**

Insulin quantification was carried out using the Bradford method. Based on measurements using UV-Vis spectroscopy, a calibration curve was plotted for a series of solutions with known protein concentrations, which allowed for determination of the amount of protein attached to the silica carriers. The quantification of the insulin present in the system, carried out after the attachment experiment, indicated the insulin content per 1 mg of the carrier-protein system, as shown in the table below **(Table 2).**

**Table 2.** Data obtained using the Bradford method describing the amount of insulin per 1 mg of the carrier-protein system

| Material | Amount of attached insulin contained in 1 mg of sample |
|---|---|
| $SiO_2$-$NH_2$-INS-E | 329 $\mu$g |
| $SiO_2$-$NH_2$-INS-C | 329 $\mu$g |

The Bradford assay results for insulin content in the systems following either electrostatic immobilisation ($SiO_2$-$NH_2$-INS-E) or covalent immobilisation ($SiO_2$-$NH_2$-INS-C) indicate effective attachment of insulin to silica particles in equal amounts for both approaches used, i.e. 329 $\mu$g of protein in 1 mg of sample, equivalent to the yield of 32.9%.

**Characterisation of surface layers of carrier-protein systems using X-ray photoelectron spectroscopy (XPS)**

To gain better insight into the properties of the carrier-protein system, the particles obtained as a result of insulin functionalisation were subjected to X-ray photoelectron spectroscopy (XPS). Analysis of the signals contained in the spectra **(Fig. 2)** obtained for silica particles and carriers with electrostatically/covalently attached insulin allowed determination of the composition of the surface layers of the samples. The results obtained are summarised in the table below **(Table 3).**

**Table 3.** Surface composition of the analysed samples based on XPS analysis

| Composition (%) | O 1s | N 1s | C 1s | S 2p | Si 2p |
|---|---|---|---|---|---|
| $SiO_2$-$NH_2$ | 77.8 | 3.6 | 6.8 | 0 | 11.8 |
| $SiO_2$-$NH_2$-INE-E | 31.9 | 13.7 | 34.5 | 1.5 | 18.3 |
| $SiO_2$-$NH_2$-INE-C | 40.2 | 7.6 | 24.2 | 0.7 | 27.3 |

The higher percentage of atoms of elements abundant in insulin, i.e. nitrogen (13.7% to 7.6%), carbon (34.5% to 24.2%) and sulphur (1.5% to 0.7%), for the electrostatically attached hormone system than for the carriers with covalently attached protein indicates a higher proportion of insulin in the surface layers of the $SiO_2$-$NH_2$-INS-E carriers than in the case of $SiO_2$-$NH_2$-INS-C. This conclusion is also confirmed by the higher proportion of silicon (27.3% to 18.3%) and oxygen (40.2% to 31.9%) building up the silica structure for $SiO_2$-$NH_2$-INS-C than for $SiO_2$-$NH_2$-INS-E.

**Study of the morphology of $SiO_2$-$NH_2$ particles and carriers with attached insulin with scanning electron microscopy (SEM)**

In order to extend the carrier characterisation, silica particles and carriers with electrostatically or covalently attached insulin were observed by scanning electron microscopy (SEM). The obtained microphotographs **(Fig. 3)** show structures in two size groups - larger ones, with diameters of about 400-500 nm, and smaller ones, with diameters of about 150-200 nm. The SEM images also reveal the spherical shape of the particles and their porous, uneven surface. No significant differences in morphology were observed for systems with or without protein, which may be related to the small size of insulin molecules, which, even when attached to silica particles, may not generate noticeable changes.

In addition to the results in **Table 3,** based on the surface nature of the XPS analysis (depth of penetration to a maximum of several nm) and knowledge of the size and morphology of the carriers, it can be concluded that biomolecules more readily localise to the surface of the silica carrier during electrostatic attachment. In contrast, 'forced' by EDC/NHS initiators to form covalent bonds, insulin molecules may localise in the pores of silica particles, thus not contributing to the surface composition (as shown by XPS analysis).

**Thermogravimetric (TG) analysis as an additional method to verify insulin attachment efficiency**

Thermogravimetric (TG) analysis was used to provide additional verification regarding the attachment efficiency of the protein to the $SiO_2$-$NH_2$ silica carriers. This technique allowed to estimate the yield of loading of the particles with protein. The values determined from the TG profiles obtained **(Fig. 4)** are summarised in the following table **(Table 4)**

**Table 4.** Data for carriers obtained from TG analysis

| Material | Mass loss [%] | INS loading [%] |
|---|---|---|
| $SiO_2$-$NH_2$ | 20.2 | - |
| $SiO_2$-$NH_2$-INE-E | 52.1 | 31.9 |
| $SiO_2$-$NH_2$-INE-C | 49.6 | 29.4 |

After comparing mass loss for silica ($SiO_2$-$NH_2$) and systems with electrostatically ($SiO_2$-$NH_2$-INE-E) or covalently ($SiO_2$-$NH_2$-INE-C) attached protein, the percentage loading of particles with insulin was calculated. The values obtained, at approximately 30% for both protein-carrier systems, are similar to each other and differ by 2.5% in favour of the system with electrostatic attachment. The data obtained by the thermogravimetric method proved to be very close to the amount of insulin carried by the particles as determined by the Bradford method, which showed an equal loading yield of 32.9%for both attachments.

**Example 3: Characterisation of the hybrid systems**

**Swelling ratio studies**

An unexpected technical feature of the freeze-dried formulation obtained is its ability to reduce swelling, manifested by low values of the experimentally determined swelling ratio ($SR$%) relative to control hydrogel materials. For all the systems obtained, a swelling experiment was conducted to estimate changes in the weight of the systems after their 24-hour exposure to a phosphate buffer environment of pH=7.4. For this purpose, the freeze-dried/hydrogel materials obtained were placed in a 24-well plate. Each sample was then covered with 1 mL of PBS solution (pH=7.4). The plate with the materials prepared in this way was incubated for 24 hours in an incubator at 37°C in slow shaking mode (50 rpm). After 24 hours, the PBS solution was drawn off and the swollen samples were rinsed three times with deionised water and weighed ($M_s$). The hydrogel materials were then dried during freeze-drying and weighed again ($M_{d0}$). The $M_{d0}$ value for freeze-dried materials was considered to be the weight of the materials placed in the wells of the 24-well plate prior to adding the PBS solution. The swelling ratio ($SR$%) was calculated according to equation (1):

$$SR\% = \frac{M_s - M_{d0}}{M_{d0}} \cdot 100\% \quad \text{(equation 1)}$$

The experiment was repeated three times and the results presented are the mean $\pm$ SD. The materials in the freeze-dried flake group are characterised by swelling ratio values in the range 908-972%, while this parameter for hydrogel materials is 6913-7343%.

Unexpectedly, after the freeze-drying process for flakes (ctrl-L, LE400, LC400,LE800, LC800), an almost eightfold reduction in the swelling of these materials is observed when the measured values are compared with the data for the injectable systems (ctrl-H, HE400, HC400,HE800, HC800).

Detailed results are shown in **Table 5** and **Fig. 5.** A swelling experiment was performed for all the materials obtained to estimate changes in the weight of the systems after their 24-hour exposure to a phosphate buffer environment of pH=7.4.

**Table 5.** Swelling ratio (SR%) values determined for the materials tested

| Material | Swelling ratio [%] |
|---|---|
| ctrl-H | 8264 |
| HE 400 | 7343 |
| HC 400 | 7352 |
| HE 800 | 7689 |
| HC 800 | 6913 |
| | |
| ctrl-L | 1219 |
| LE 400 | 929 |

(continued)

| Material | Swelling ratio [%] |
|---|---|
| LC 400 | 908 |
| LE 800 | 974 |
| LC 800 | 972 |

In the case of injected materials **(Fig. 5,** left side), no significant differences are observed in the swelling ratio values obtained. The values calculated for systems that differ in particle content (HE400 and HC400 vs. HE800 and HC800) or in the type of protein binding in the injected carriers (HE400 and HE800 vs. HC400 and HC800) do not differ significantly from the hydrogel control without silica structures (ctrl-H). Similar conclusions can be drawn for a series of materials based on freeze-dried flakes **(Fig. 5,** right side). In this group too, neither the type of insulin binding (LE400 and LE800 vs. LC400 and LC800) nor the content of particles attached to the biopolymer matrix (LE400 and LC400 vs. LE800 and LC800) lead to statistically significant differences in the swelling ratio compared to the control without carriers (ctrl-L). Notably, an almost eightfold reduction in swelling is observed for the flakes (ctrl-L, LE400, LC400, LE800, LC800) after the freeze-drying process when the measured values are compared with the data for the injectable systems (ctrl-H, HE400, HC400, HE800, HC800).

### Aqueous phase content ($f_{aq}$) for hybrid materials in the form of lyophilisates

Based on the results of the swelling experiment conducted under physiological conditions (described above), it was also possible to determine the aqueous phase content ($f_{aq}$) per mg of dry sample for the hybrid materials in freeze-dried form. This is because for this group of materials, the mass of the materials immediately after the freeze-drying process (dry flakes) was considered as the initial mass ($M_{d0}$). The mass of the aqueous phase ($M_{aq}$) that the material was able to absorb after 24-hour incubation in PBS was calculated as the difference between the swollen material ($M_s$) and the dry flake ($M_{d0}$). The values obtained are summarised in **Table 6.**

**Table 6**. Aqueous phase content $f_{aq}$ (mg) for the freeze-dried materials

| Material | Aqueous phase content ($f_{aq}$, mg) |
|---|---|
| LE 400 | 9.3 |
| LC 400 | 9.1 |
| LE 800 | 9.7 |
| LC 800 | 9.7 |

Comparing the obtained $f_{aq}$ values (9.1-9.7 mg), it can again be concluded that both the type of insulin attachment and the content of the particles introduced into the matrix do not generate significant differences in the ability of this group of materials to absorb the aqueous phase.

### Degradation of selected systems under artificial saliva conditions

Due to the fact that the developed materials are intended specifically for use as dental materials, an additional degradation experiment was carried out under artificial saliva conditions. This experiment was carried out for the injectable systems with a lower concentration of particles with two different insulin attachment types (HE400 and HC400) and the freeze-dried systems with a higher concentration of carriers (LE800 and LC800) The results obtained from the analysis are summarised in the accompanying graph **(Fig. 6).**

After the study, it was noted that all of the materials belonging to the series of injectable systems (ctrl-H, HE400 and HC400) kept their mass relatively constant and no differences were observed due to the type of protein binding. In all the lyophilisates tested (ctrl-L, LE400 and LC400), there is no loss in the mass of the systems - on the contrary, often a slight increase. The situation described is closely related to the nature of the flake-like materials, which first swell from their light, dry form, leading to an increase in their weight due to the absorbed fluid. In contrast, artificial saliva, acting as a degradation environment, proved incapable of weakening the structure of any of the systems tested sufficiently to lead to their disintegration and consequent loss of mass.

**Insulin release from the model injectable and lyophilisate systems and the encapsulation efficiency in gel**

Given the limited detection level of insulin using the Bradford method, it was decided to perform the release of the hormone from model systems representing the materials described above. Materials with a threefold higher particle concentration than the higher ones used so far (i.e. 0.8 mg of carrier-protein system in the sol) were used as model systems. The hybrid injectable systems containing particles with insulin attached electrostatically (HE) or covalently (HC) and analogous samples from the lyophilisate group enriched with two types of carrier with protein bound covalently (LC) or via electrostatic interaction with $SiO_2$-$NH_2$ (LE) were tested. The encapsulation efficiency (EE) of the hormone particles in the biopolymer matrix was also determined for the model systems studied, also assayed by the Bradford method based on the theoretical insulin content within the system and quantification of the unencapsulated protein remaining in the aqueous phase after the cross-linking process. The EE values obtained, in the range of 88-94% for all materials obtained, are summarised in the table below **(Table 7).**

Table 7. Encapsulation efficiencies determined for the model systems for which the release was carried out

| Material | Encapsulation efficiency [%] |
|----------|------------------------------|
| HE | 94±4 |
| HC | 88±1 |
| LE | 93±3 |
| LC | 91±1 |

The cumulative percentage of insulin released from the hybrid materials is shown in the accompanying graph **(Fig. 7).** The experiment showed a rapid release of approximately 40% of the protein from the injectable materials, with no differences due to the type of binding of the hormone molecules to $SiO_2$-$NH_2$ (HE and HC), after 6 hours. In contrast to hydrogel systems, for lyophilisates at the same time point (after 6 hours), about 2% of protein was released from particles with covalently bound insulin (LC) and 23% for the counterpart with electrostatic attachment (LE). A plausible explanation for the higher amounts released for the injectable series of materials is that within the hydrogel network of HE and HC materials, i.e. systems with a larger initial volume, there may have been easier diffusion of INS, especially from the surface layers of these materials whereas for the lyophilisates the first hours of the experiment led to their swelling first. At subsequent time points from 24 to 72 hours, a rather steady, non-rapid increase in released insulin is observed - the fastest from 60 to 92% for HE, followed by 64 to 87% for HC and slower from 47 to 59% for the LE system and the slowest from about 3% to about 8% for LC. Complete insulin release occurs after 144 hours only for the injectable material with particles carrying electrostatically-bound insulin (HE). For the last timepoint considered in the experiment (after 144 hours), insulin was released in 92% from the HC system, 70% from LE and 12% from LC. It can therefore be concluded that both the type of protein binding (covalent/electrostatic) and the final form of the material (hydrogel/lyophilisate) influence the kinetics of hormone release. A trend that is true for both groups of materials is observed, that covalent binding limits the rate of protein release from the system. Furthermore, when comparing the release profiles for the corresponding materials from the two different groups, but containing carriers with identically bound insulin (HE vs LE and HC vs LC), a significant delay in release is observed for the freeze-dried materials.

**CONCLUSIONS:**

In conclusion, the physicochemical studies allowed the following systems to be selected for further *in vitro* biological analysis. Given the slowed release of insulin from the hybrid systems in freeze-dried form, materials containing higher concentrations of insulin carriers (LE800 and LC800) were chosen to increase the chances of inducing therapeutic effects in cell experiments. Among the injectable hybrid systems, a more economical version was selected in the form of systems with lower particle content (HE400 and HC400).

**Example 4: Biological studies**

**II. *In vitro* biological assays**

**1. Insulin-containing materials tested do not release harmful degradation products**

Local administration of hydrogel materials or lyophilisates results in their slow degradation in the body. The degradation products of the materials, together with the interstitial fluid, enter the bloodstream. This can result in a systemic reaction to the topically administered material. The most sensitive to such degradation products are blood cells including erythrocytes, neutrophils or mononuclear cells (mainly lymphocytes and monocytes) and liver cells - hepatocytes. Therefore, we performed an in-depth analysis of the toxicity of the degradation products of the tested hydrogels and lyophilisates with insulin. Extracts containing degradation products secreted into PBS were collected from hydrogels (ctrl-H, HE400 and HC400) and lyophilisates (ctrl-L, LE800 and LC800) after 1, 2, 3 or 6 days of incubation at 37°C.

The results confirm the absence of haemolytic properties of the degradation products resulting from the degradation of both hydrogels and lyophilisates **(Fig. 8).** The level of haemolysis, directly proportional to the absorbance value, obtained for all the extracts tested is the same as for the control (PBS).

Subsequently, the viability of PMN cells (neutrophils) and PBMC (mononuclear cells) isolated from human peripheral blood was examined after incubation with the obtained extracts. The results show a slight reduction in the viability of PMN cells incubated with extracts collected from the ctrl-L or HC400 after 1 day. However, other extracts collected at other time points did not decrease viability of neither PMN nor PBMC cells **(Figs. 9 and 10).**

The viability of HegG2 cells (human hepatocyte model) was then analysed. Liver cells are an important element in the detoxification process of xenobiotics, understood as chemicals that are not natural components of a living organism. Therefore, harmful degradation products of the materials represent a potential cytotoxic agent for these cells. In this study, extracts obtained from ctrl-H, HE400 and HC400 showed a moderate toxic effect against HepG2 cells after 1 day of hydrogel degradation - reducing their viability to about 60%. The other extracts obtained were characterised by a lack of effect on HepG2 cell viability **(Fig. 11).**

<u>Summary 1</u>: The extracts obtained from the hydrogel-insulin system show slight toxicity towards human neutrophils and human liver cells. However, this effect applies to extracts obtained only after 24 hours and is observed for both control and insulin-containing hydrogel. Therefore, this effect is independent of the insulin attached to the hydrogel. At the same time, the extracts obtained from the lyophilisate-insulin system are completely inert with respect to the normal cell models tested.

## 2. The tested materials and their degradation products are not toxic to bone-forming nor bone-resorbing cell precursors.

During the course of osteoporosis, the lack of toxicity of insulin-containing materials towards bone-forming cell precursors is important. In this project, human mesenchymal stem cells (hBMMSCs) were used to assess the safety of the tested materials and their degradation products (present in extracts collected from the materials). The attachment of insulin to the hydrogel materials or lyophilisates did not significantly affect the viability of the hBMMSC cells, demonstrating its lack of toxicity. Some HC400 and LC400 materials (after 3 or 7 days of culture) induced an increase in hBMMSC cell viability to approximately 120-140% of control (ctrl-H or ctrl-L) **(Fig. 12).** However, the results of the proliferation assay of hBMMSC cells incubated on ctrl-L, LE800 and LC800 indicate that these materials have no effect on cell numbers. In addition, incubation of hBMMSCs with extracts collected from ctrl-H, HE400 or HC400 shows a slight decrease in the proliferation level of hBMMSC cells **(Fig. 13).**

Insulin as a growth factor can also alter the viability of bone-resorbing cell precursors. RAW 264.7 mouse monocyte-macrophage cell line was used as a model for such cells. The results obtained show that the tested materials, both the hydrogel materials and lyophilisates with insulin, do not affect the viability of RAW 264.7 cells **(Fig. 14).** Moreover, the lyophilisates with insulin also do not affect the proliferation level of these cells. Extracts harvested from ctrl-H, HE400 or HC400 inhibit the proliferation of RAW 264.7 cells **(Fig. 15).** This applies to extracts collected after 1 day and is not related to the presence of insulin in these preparations, as the effect is also observed for extracts derived from the control material (ctrl-H).

<u>Summary 2</u>: The insulin-containing materials tested are not toxic to bone-forming/osteoblast and bone-resorbing/osteoclast cell precursors. The results also indicate that the tested materials, both hydrogels and lyophilisates with insulin, stimulate moderately the metabolic activity of hBMMSC cells, but do not affect the proliferation level of these cells.

## 3. The tested materials or their degradation products increase the expression of osteoblast markers while not inducing the differentiation process of bone-resorbing cells.

The regeneration process of bone defects is preceded by the differentiation of precursor cells towards osteoblasts. One sign of the differentiation process is an increase in the activity of the marker enzyme - alkaline phosphatase (ALP). The results show that both incubation of hBMMSC cells with extracts obtained from ctrl-H, HE400 or HC400 and incubation directly with lyophilisates ctrl-L, LE800 do not affect ALP activity in hBMMSC cells. An increase in ALP activity was observed after a 3-day incubation with LC800 **(Fig. 16).**

Subsequently, the expression of genes that are activated during the differentiation process of mesenchymal cells towards bone-forming cells was analysed. The following genes were selected for analysis: 1) human alkaline phosphatase (hALP); 2) osteopontin (hOP), which is an extracellular protein whose high levels are observed during the onset of mineralisation; 3) osteocalcin (hOCN), a marker that appears at a late stage of osteoblast differentiation, along with mineralisation; and 4) human transcription factor hRunx2, which is activated at the beginning of the osteoblast differentiation process. An experiment designed in this way allowed for tracking expression levels of genes activated at different stages of the bone formation process. After 7 days of culturing hBMMSC cells on HE400 and LE800 insulin-containing materials, an increase in the level of an early marker of the bone formation process, the transcription factor hRunx2, was observed **(Fig. 17**A and C). Next, on day 14 of hBMMSC culture, the expression level of hRunx2 decreased to the value obtained for insulin-free materials **(Fig. 17**B and D). There was also an increase in the expression of genes important for later stages of osteoblast differentiation. HE400 and HC400 insulin-containing hydrogels induced an approximately 1.5-fold increase in hOCN levels after 7 days of hBMMSC culture, which then increased to approximately 3-fold levels after 14 days of culture **(Fig. 17**A and B). LE800 lyophilisates with insulin induced an approximately 2-fold increase in hOP and a 3-fold increase in hOCN on day 7 of culture of hBMMSC cells. However, these levels declined on day 14 of culture to values obtained for insulin-free materials. The least effect on the expression of genes involved in bone formation was observed for LC800 insulin-containing lyophilisates. For this type of material, an approximately 1.75-fold increase in hOP levels was shown on day 7 of the cell culture, which then decreased on day 14 of culture to the control value (obtained for ctrl-L) **(Fig. 17**C and D).

In addition, the presence of calcium phosphate crystals, which are the final stage of maturing osteoblasts leading to bone mineralisation, was examined. However, it was not observed that the tested extracts collected from hydrogels with insulin or the lyophilisates with insulin stimulated hBMMSC cells towards the mineralisation process **(Fig. 18)**.

The effect of insulin-containing materials on the differentiation of RAW 264.7 cells towards osteoclasts was also investigated. This time, the activity of tartrate-resistant acid phosphatase (TRAP), a marker of bone-resorbing cell differentiation, was determined. The results indicate that the tested materials with insulin do not stimulate the differentiation of RAW 264.7 cells towards osteoclasts. In the case of extracts collected from ctrl-H, HE400 or HC400 hydrogels, a decrease in TRAP enzyme activity relative to control cells was even observed. This phenomenon applies to both the control hydrogel and those containing insulin, so insulin did not contribute to the decrease in TRAP activity. A slight increase in TRAP enzyme activity was observed after a 3-day incubation of RAW 264.7 cells with LE800 **(Fig. 19)**. However, in no case was osteoclast-specific cell morphology observed (results not shown).

The levels of genes responsible for the differentiation of RAW 264.7 cells towards osteoclasts were also determined **(Fig. 20)**. Insulin can also affect osteoclast precursors, so it was verified whether the tested materials affect the differentiation of RAW 264.7 cells. Compared to insulin-free material (ctrl-H or ctrl-L), only a slight increase in the transcription factor mNfatc1 was observed after a 3-day culture of RAW 264.7 cells on HE400 hydrogels. A decrease in markers (mCtsK, mNfatc1, mDC-STAMP) indicative of the differentiation process of RAW 264.7 cells towards osteoclasts was observed with HC400 hydrogel. A decrease in the mNfatc1 gene expression level was also observed for LC800 insulin-containing lyophilisates. Such observations indicate that the attachment of insulin to the tested materials inhibits the expression of genes involved in the differentiation process of osteoclast precursors. Summary 3: Culture of hBMMSC cells on LC800 lyophilisates with insulin results in short-term elevated ALP enzyme activity characteristic of the differentiation process towards osteoblasts. In addition, for LE800 and LC800 lyophilisates, a moderate increase in osteopontin and osteocalcin genes, which are markers of osteoblasts, was shown to persist until day 7 of culture. For the other hydrogel materials with insulin HE400 and HC400, an increase was shown primarily in the expression level of osteocalcin. Elevated expression levels of this gene persisted even on day 14 of culture of hBMMSC cells on HE400 and HC400 hydrogels. However, no signs of a mineralisation process were observed after incubation of hBMMSC cells with the insulin-containing materials tested. The tested materials did not stimulate the differentiation process of RAW 264.7 cells towards osteoclasts. In the case of HC400 hydrogel and LC800 lyophilisate, a decrease in markers of the differentiation process of RAW 264.7 cells towards osteoclasts was even observed.

**CONCLUSION:** The tested hydrogel materials as well as the lyophilisates with insulin are safe to the normal cell models studied. Performing such a thorough analysis of cytotoxicity in preclinical *in vitro* studies qualifies the indicated materials for animal model studies. The effect of insulin in HE400, HC400, LE800 and LC800 materials on the increased expression of markers indicative of hBMMSC differentiation towards osteoblasts was demonstrated. However, this process was stopped at the stage of early osteoblast maturation. Incubation of hBMMSCs with HE400, HC400, LE800 and LC800 does not lead to a mineralisation process - the formation of calcium phosphate crystals. Insulin immobilised in the different materials showed a different effect in terms of induction of genes responsible for the bone-forming process. Insulin in HC400 and LC800 materials had an inhibitory effect on the expression of osteoclast markers. This phenomenon indicates that the therapeutic effect of insulin in the materials may also be related to a decrease in the activity of cells responsible for the resorption process. The analyses using qRT-PCR showed an increase in the transcription factor hRunx2 in hBMMSCs after incubation with the insulin-containing materials tested. The hRunx2 transcription factor is a component of the signal transduction pathway from insulin. Therefore, this result confirms that insulin immobilised in the materials is active.

**Claims**

1. A composition comprising:

   - a polymer matrix comprising: collagen (Coll), chitosan (Ch) and hyaluronic acid functionalized with amine groups (HAmod), preferably with a Col:Ch:HAmod weight ratio of 50:20:30 and dispersed therein
   - silica particles with insulin attached to their surface,

   whereby it has the form of a lyophilized hydrogel.

2. The composition of claim 1, **characterised in that** it obtains a swelling ratio (SR) of not more than 1000% after incubation in PBS phosphate buffer of pH=7.4 at 37°C for 24 hours and subsequent rinsing with water, the swelling ratio being calculated with the following formula:

$$SR\% = \frac{M_s - M_{d0}}{M_{d0}} \cdot 100\%$$

   where:

   $M_S$ is the weight of the composition after incubation in phosphate buffer,
   $M_{d0}$ is the dry weight of the composition.

3. The composition of claim 1, **characterised in that**, after incubation in PBS phosphate buffer pH=7.4 at 37°C for 24 hours, the content of the aqueous phase does not exceed 10 mg per 1 mg dry weight of the composition, preferably lying in the range 9.1-9.7 mg.

4. A method for the preparation of a composition comprising a polymeric matrix comprising collagen (Col), chitosan (Ch) and hyaluronic acid functionalized with amine groups (HAmod) and dispersed therein silica particles with insulin attached to their surface, **characterized in that**:

   a) an aqueous dispersion of silica particles with insulin attached to their surface is obtained,
   b) solutions of collagen (Col), chitosan (Ch) and hyaluronic acid functionalized with amine groups (HAmod) are obtained,
   c) the dispersion obtained in step a) is mixed with a mixture of the solutions obtained in step b) and a genipin solution, preferably using a mixture of the solutions obtained in step b) with a Col:Ch:HAmod weight ratio of 50:20:30,
   d) the sol obtained in step c) is left to crosslink, preferably for 48 hours at 37°C,
   e) the hydrogel obtained in step d) is purified and lyophilised.

5. The method of claim 4, **characterised in that** in step a) a dispersion of silica particles with insulin attached in an electrostatic manner (SiO$_2$-NH$_2$-INS-E) is obtained.

6. The method of claim 4, **characterised in that** in step a) a dispersion of silica particles with insulin attached in a covalent manner (SiO$_2$-NH$_2$-INS-C) is obtained.

7. The method of claim 4, **characterised in that** in step a), using water as a dispersion medium, a dispersion of silica particles with insulin is obtained containing 4 mg/mL to 8 mg/mL of the silica particles with insulin.

8. The method of claim 7, **characterised in that** the size of the silica particles is between 100 nm and 500 nm.

9. The method of claim 7, **characterised in that** the degree of insulin loading of the silica particles with insulin is from 30 to 35% by weight when the amount of insulin is measured by the Bradford method.

10. The method of claim 4, **characterised in that** in step b) a collagen solution at a concentration of 3.42-4.06 mg/mL in HCl at a concentration of 0.01 mol/dm$^3$ is used.

11. The method of claim 4, **characterised in that** in step b) a solution of chitosan at a concentration of 1% (w/w) in 1%

CH$_3$COOH is obtained.

12. The method of claim 4, **characterised in that** in step b) a solution of functionalized hyaluronic acid at a concentration of 1% (w/w) in 10x PBS is obtained.

13. The method of claim 4, **characterised in that** in step c) a solution of genipin at a concentration of 18 mg/mL in 96% EtOH is used.

14. The method of claim 4, **characterised in that** in step e) additionally the obtained hydrogel is washed, preferably with deionised water, frozen and freeze-dried.

15. The method of claim 14, **characterised in that** freeze-drying is carried out at -52°C, under reduced pressure, in order to remove solvents.

**Fig. 1**

**Fig. 2**

**Fig. 3**

**Fig. 4**

**Fig. 5**

**Fig. 6**

**Fig. 7**

Fig. 8

Fig. 9

Fig. 10

Fig. 11

Fig. 12

**Fig. 13**

**Fig. 14**

Fig. 15

hBMMSC

Fig. 16

Fig. 17

14th day of cell culture in the presence of extracts collected from above the hydrogels

21st day of cell culture on the material

Fig. 18

RAW 264.7

A

B

Fig. 19

RAW 264.7

**A** 3rd day of cell culture on the material

Fig. 20

Europäisches Patentamt

European Patent Office

Office européen des brevets

# EUROPEAN SEARCH REPORT

**Application Number**

EP 24 46 1623

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KRAJCER ALEKSANDRA ET AL: "Bioactive injectable composites based on insulin-functionalized silica particles reinforced polymeric hydrogels for potential applications in bone tissue engineering", JOURNAL OF MATERIALS SCIENCE & TECHNOLOGY, ALLERTON PRESS, AMSTERDAM, NL, vol. 105, 16 September 2021 (2021-09-16), pages 153-163, XP087010736, ISSN: 1005-0302, DOI: 10.1016/J.JMST.2021.08.003 [retrieved on 2021-09-16] * "2.2.1. Electrostatic/covalent insulin immobilization to SiO2-NH2 particles"; page 154 * * "2.2.2. Composite materials synthesis"; page 155 * * "2.4.2 Thermogravimetry measurements (TG)"; page 155 * * abstract * * figure 5 * * "3.1.1 Verification of electro- and covalent insulin immobilization"; page 156 * | 1-15 | INV. A61L27/44 A61L27/52 A61L27/54 |
|  | ----- -/-- |  | **TECHNICAL FIELDS SEARCHED (IPC)** A61L |

The present search report has been drawn up for all claims

| Place of search | Date of completion of the search | Examiner |
|---|---|---|
| The Hague | 4 February 2025 | Zalfen, Alina |

EPO FORM 1503 03.82 (P04C01)

Europäisches
Patentamt

European
Patent Office

Office européen
des brevets

# EUROPEAN SEARCH REPORT

**Application Number**

EP 24 46 1623

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LEWANDOWSKA-LANCUCKA JOANNA ET AL: "Genipin crosslinked bioactive collagen/chitosan/hyaluronic acid injectable hydrogels structurally amended via covalent attachment of surface-modified silica particles", INTERNATIONAL JOURNAL OF BIOLOGICAL MACROMOLECULES, ELSEVIER BV, NL, vol. 136, 25 June 2019 (2019-06-25), pages 1196-1208, XP085753760, ISSN: 0141-8130, DOI: 10.1016/J.IJBIOMAC.2019.06.184 [retrieved on 2019-06-25] * abstract * * "2.3.2 Hybrid materials characterization"; page 1196, right-hand column * | 1-15 | |
| A | GILARSKA ADRIANA ET AL: "Collagen/chitosan/hyaluronic acid - based injectable hydrogels for tissue engineering applications - design, physicochemical and biological characterization", COLLOIDS AND SURFACES B: BIOINTERFACES, ELSEVIER AMSTERDAM, NL, vol. 170, 4 June 2018 (2018-06-04), pages 152-162, XP085458741, ISSN: 0927-7765, DOI: 10.1016/J.COLSURFB.2018.06.004 * 2nd paragraph; page 153, left-hand column * * "2.2.1 Hydrogel scaffolds preparation"; page 153, right-hand column * * "2.2.3 Swelling properties"; page 154, left-hand column * | 1-15 | TECHNICAL FIELDS SEARCHED (IPC) |

-/--

The present search report has been drawn up for all claims

| Place of search | Date of completion of the search | Examiner |
|---|---|---|
| The Hague | 4 February 2025 | Zalfen, Alina |

Europäisches
Patentamt
European
Patent Office
Office européen
des brevets

## EUROPEAN SEARCH REPORT

**Application Number**

EP 24 46 1623

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2020/171723 A2 (UNIV JAGIELLONSKI [PL]) 27 August 2020 (2020-08-27) * claims 1,3,6,9 * | 1-15 | |
| A | EP 2 529 764 A1 (CURASAN AG [DE]) 5 December 2012 (2012-12-05) * claims 1,8,9,15,29 * | 1-15 | |

TECHNICAL FIELDS
SEARCHED     (IPC)

The present search report has been drawn up for all claims

| Place of search | Date of completion of the search | Examiner |
|---|---|---|
| The Hague | 4 February 2025 | Zalfen, Alina |

EPO FORM 1503 03.82 (P04C01)

## ANNEX TO THE EUROPEAN SEARCH REPORT
## ON EUROPEAN PATENT APPLICATION NO.

EP 24 46 1623

This annex lists the patent family members relating to the patent documents cited in the above-mentioned European search report.
The members are as contained in the European Patent Office EDP file on
The European Patent Office is in no way liable for these particulars which are merely given for the purpose of information.

04-02-2025

| Patent document cited in search report | | Publication date | Patent family member(s) | | Publication date |
|---|---|---|---|---|---|
| WO 2020171723 | A2 | 27-08-2020 | EP | 3927387 A2 | 29-12-2021 |
| | | | ES | 2973340 T3 | 19-06-2024 |
| | | | PL | 239179 B1 | 08-11-2021 |
| | | | US | 2022047776 A1 | 17-02-2022 |
| | | | WO | 2020171723 A2 | 27-08-2020 |
| EP 2529764 | A1 | 05-12-2012 | CN | 104039367 A | 10-09-2014 |
| | | | DK | 2714112 T3 | 08-01-2018 |
| | | | EP | 2529764 A1 | 05-12-2012 |
| | | | EP | 2714112 A2 | 09-04-2014 |
| | | | HR | P20180030 T1 | 23-02-2018 |
| | | | HU | E035344 T2 | 02-05-2018 |
| | | | JP | 6162106 B2 | 19-07-2017 |
| | | | JP | 2014515966 A | 07-07-2014 |
| | | | JP | 2017124207 A | 20-07-2017 |
| | | | KR | 20140040172 A | 02-04-2014 |
| | | | KR | 20190105110 A | 11-09-2019 |
| | | | LT | 2714112 T | 10-01-2018 |
| | | | PL | 2714112 T3 | 30-03-2018 |
| | | | PT | 2714112 T | 22-12-2017 |
| | | | US | 2014170202 A1 | 19-06-2014 |
| | | | WO | 2012163532 A2 | 06-12-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2396046 A
- US 20220111119 A
- EP 2956185 A
- EP 2529764 A

**Non-patent literature cited in the description**

- Bioactive injectable composites based on insulin-functionalized silica particles reinforced polymeric hydrogels for potential applications in bone tissue engineering. *Journal of Materials Science & Technology*, 2022, vol. 105
- **KRAJCER A et al.** *Journal of Materials Science & Technology*, 2022, vol. 105, 153-163
- **GILARSKA A et al.** *Int. J. Biol. Macromol*, 2020, vol. 155, 938-950